# EUROPEAN PATENT APPLICATION

(11) **EP 1 097 700 A1**
(43) Date of publication of application: **09.05.2001**
(21) Application number: 00925619.9
(22) Date of filing: 11.05.2000
(51) Int. Cl.: A61K 7/06

(54) **HAIR GROWTH STIMULANTS**

(30) Priority: 14.05.1999 JP 13413299
(71) Applicant: Japan Applied Microbiology Research Institute Co., Ltd., Higashi-Yatsuhiro-gun, Yamanashi-ken 406-0045 (JP)
(72) Inventor: HORIUCHI, Isao, 1014-banchi, Yamanashi-ken 405-0056 (JP)
(74) Representative: Bubb, Antony John Allen
(86) International application number: JP0003017
(87) International publication number: WO0069399

(57) **Abstract**

A hair growth tonic contains, as an active ingredient, a filtrate of lactic acid bacterial culture such as Streptococcus lactis, and Lactobacillus bulgaricus. The use of this type of hair growth tonic promotes hair regeneration, hair growth and hair nourishment.

## Description

### TECHNICAL FIELD

The present invention relates to a hair growth tonic. More specifically, it relates to a growth tonic for hair, which contains the filtrate of lactic acid bacterial cultures as its active ingredient.

### BACKGROUND ART

A hair consists of a hair shaft that sticks out from the skin surface and a hair root that is inside the skin. The hair root is encapsulated in a hair follicle. The hair root further contains the hair bulb and a hair papilla. The hair papilla has capillaries and nerves, and regulates the formation and growth of the hair through uptake of nutrients from food and oxygen. At a location close to the hair papilla, there are hair matrix cells that generate the hair. In other words, the hair matrix cells take in nutrients and oxygen from the capillaries that enter the hair papilla and hair is formed by repeated division of the cells.

Each hair has an independent lifecycle. Hairs, as a whole, repeat the cycle of growth, falling off, and generation of new hair. In fact, the hair follicle has a hair cycle of growth phase (anagen), regressive phase (catagen) and a resting phase (telogen). Hair is produced during the growth phase only. During this period, the hair papilla is large, the hair matrix cells work actively to extend the hair, and the hair bulb extends up to the subcutaneous tissue. Once the growth stops, the hair follicle passes through a regressive phase. The first symptom of the regressive phase is the stoppage of melanin production by the hair bulb. After then, most of the other cells of the hair follicle are consumed by the surrounding micropahges to shrink. The hair root shrinks up to a position below the arrector pili muscle and the cells enter to the resting phase. Generally, the growth phase of a hair is 5-6 years, the regressive phase thereof is 2-3 weeks and the resting phase thereof is 2-3 months.

When there is some abnormality during the hair cycle, it falls off and baldness starts. The causes of baldness are not fully understood. The lowering of hair follicle function under the influence of the male hormone, the lowering of the metabolic functions of the hair follicle and bulb, the lowering of the physiological functions of the scalp, some localized problem in blood flow due to tension on the scalp, malnutrition, stress, side effects of drugs, heredity, etc could be the reason. Various types of hair growth tonics have been used for preventing and treating baldness. Such hair growth tonics are mostly mixtures of various chemicals that have some effect of counteracting at least some of the causes listed above. The main component of most of these tonics is a chemical that either has an activating effect on the hair matrix cells or a blood circulation enhancing effect. Most of these tonics are designed to give a hair growth promoting effect (hair nourishment and hair regeneration) by supplying nutrients through the activation of the hair follicles that show diminished hair growth and through enhanced blood flow.

Hair and baldness have been discussed, for instance, in *Shin Keshohin Gaku* (New Cosmetology) compiled by Takeo Mitsui and published by Nanzando (1994), which may be referred.

Patent applications on various hair tonics and hair growth tonics have been applied for. Some examples are disclosed in Japanese Patent Laid-open Publication No. Sho. 63-303915 (A hair tonic containing skimmia extract), Japanese Patent Publication No. Hei. 4-5002 (A cosmetic hair tonic containing lipase), Japanese Patent Laid-open Publication No. Hei. 1-254614 (A hair tonic containing fermented herbal stimulant and water-soluble chitosan).

As mentioned above, various types of hair tonics and hair growth tonics have been developed. However very few of them are truly effective in suppressing hair loss or promoting hair nourishment and hair growth. Under this situation, it would be very significant to discover a hair growth tonic that is truly effective, because it would be of immense benefit for people suffering from baldness.

After extensive research undertaken to overcome the above-mentioned problems, the present inventors found that the filtrate of cultures of some microorganisms, lactic acid bacteria in particular, is effective in the treatment of baldness, and completes the present invention.

Thus, an object of the present invention is to provide a hair growth tonic that can effectively promote hair growth, hair nourishment and hair regeneration (hereinafter referred to simply as "hair growth").

### DISCLOSURE OF THE INVENTION

A hair growth tonic according to the present invention is characterized by containing filtrate of lactic acid bacterial cultures, as its active ingredient.

Moreover, a hair growth tonic according to another aspect of the present invention is characterized in that the above-mentioned lactic acid bacterium is a bacterium belonging to Streptococcus or Lactobacillus.

In addition, a hair growth tonic according to another aspect of the present invention is characterized in that the above-mentioned lactic acid bacterium of Streptococcus is Streptococcus lactis.

A hair growth tonic according to still another aspect of the present invention is characterized in that the above-mentioned lactic acid bacterium of Lactobacillus is Lactobacillus bulgaricus.

Furthermore, a hair growth tonic according to another aspect of the present invention is characterized by containing other known hair growth promoting component in addition to the filtrate of the above-mentioned lactic acid bacterial cultures.

Still further, a hair growth tonic according to another aspect of the present invention is characterized in that the above-mentioned known other growth promoting component is laurel extract and/or chlorophyll.

Because of the above-described composition, the filtrate of the lactic acid bacterial cultures have hair growth promoting as well as hair loss preventing effect. It is assumed that the components contained in the filtrate of the lactic acid bacterial cultures have the effect of promoting blood circulation to the peripheral capillaries, and also that the components enhance the division and multiplication of the hair matrix cells and act as hair root activators by improving the lowered functions of the hair matrix cells. Particularly when the lactic acid bacterium belonging to Streptococcus or Lactobacillus is used, various components produced by the lactic acid bacterium, which are present in the filtrate of the lactic acid bacterial culture and are supplied to the hair papilla, act synergistically and have the effect of promoting the growth of the hair matrix cells.

In the present invention, "filtrate of culture" means the liquid fraction of the lactic acid bacterial culture, obtained by crushing the bacterial cells present in the culture medium (bacterial suspension) to make them into colloidal or dissolved form, and the removing the cellular residue. This filtrate can be separated by solid-liquid separation means like filtration, centrifuging, etc. In one embodiment of the present invention, the lactic acid bacteria used are those belonging to Streptococcus or Lactobacillus. However, in the hair growth tonic of the present invention, it is not limited to use the filtrate of the culture of the specified lactic acid bacteria. The hair growth tonic of the present invention can contain filtrates from cultures of any other lactic acid bacteria having a similar effect.

The hair growth tonic of the present invention can contain other known hair growth promoting components. Examples of such hair growth promoting components are activators of living cells such as laurel extract, and chlorophyll; blood circulation promoters and localized stimulants that promote blood flow to peripheral capillaries; nutrients (such as Vitamins and amino acids) for supplying nutrition to the area surrounding hair matrix cells; female hormone formulations that have antagonistic effect against male hormone; hair root activators for improving the lowered function of hair matrix cells; moisturizers for preventing scalp drying, etc.

### BRIEF DESCRIPTION OF THE DRAWINGS

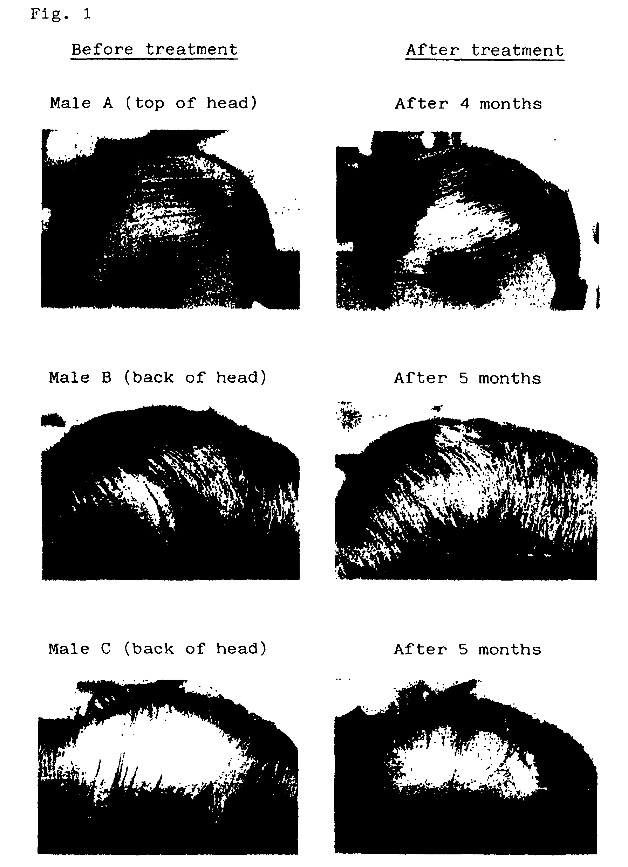

Fig. 1 shows the comparison of hair growth before the start of the test and the hair growth 4 or 5 months after treatment with the filtrate of cultures of Streptococcus lactis culture from Example 1 (Formulation 2), applied to the scalp of three males (Top: Male A, Middle: Male B and Bottom: Male C) having alopecia.

### BEST MODE FOR CARRYING OUT THE INVENTION

The hair growth tonic according to the present invention will be described in greater detail, referring to the Tables and the accompanying drawings.

"Lactic acid bacteria" is a general name for a group of bacteria that obtain their energy by fermenting carbohydrates and produce large quantities of lactic acid. Lactic acid bacteria can be classified into cocci and bacilli. Streptococcus, Leuconostoc, Pediococcus, etc. belong to the group of cocci and Lactobacillus, Vifidobacterium, etc. belong to the group of bacilli. The lactic acid bacteria in the present invention include any types of lactic acid bacteria belonging to the above-mentioned groups. Unnatural lactic acid bacteria such as recombinant lactic acid bacteria, variants of lactic acid bacteria obtained by artificially induced mutations, etc are also included. The preferred lactic acid bacteria are those which are not pathogens of humans and other animals. The commercially available lactic acid bacteria used for manufacturing foods such as fermented milk products, fermented meat products, fermented food, fermented soy bean milk, pickles, etc. can be more preferably used. Such lactic acid bacteria are available from, for example, Chr. Hansen's Corp.

Examples of lactic acid bacteria that can be used in the present invention includes Streptococcus species such as Streptococcus thermophilus, Streptococcus lactis, and Streptococcus lactis subap. Diacetilactis; Pediococcus species such as Pediococcus cerevisiae, Pediococcus acidilactici, Pediococcus penntosaceus, Pediococcus halophilus, and Pediococcus urinae-equi; Leuconostoc species such as Leuconostoc cremoris and Leuconostoc oenos; Lactobacillus species such as Lactobacillus delbrueckii, Lactobacillus leuchmannii, Lactobacillus lactis, Lactobacillus bulgaricus, Lactobacillus helveticus, Lactobacillus fermenntum, Lactobacillus brevis, and Lactobacillus viridescens; Bifidobacterium species such as Bifidobacterium longum, Bifidobacterium bifidum, Bifidobacterium breve, and Bifidobacterium infantis.

Among these lactic acid bacteria, those belonging to the genus Streptococcus are gram-positive, non-sporulating and obligate to facultative anaerobic cocci of diameter 1 µm or less. Normally, they are diplococci or streptococci. They are negative for catalase and oxidase and produce dextrorotatory lactic acid in the homofermentation mode. Those belonging to the genus Lactobacillus are gram-positive, negative for catalase, and are obligate anaerobes to anaerobes. Normally they are non-motile, do not produce spores, are bacilli, and aggregate into chains also. Both homofermentation and heterofermentation types are known to exist.

In one embodiment of the present invention, the filtrate of cultures of two of the lactic acid bacteria listed above, Streptococcus lactis and Lactobacillus bulgaricus, are used. Streptococcus lactis belongs to cocci and Lactobacillus bulgaricus is a bacillum. Both are homofermentation type lactic acid bacteria. Streptococcus lactis is used as a starter in the production of dairy products such as fermented milk, cheese, and butter. Lactobacillus bulgaricus is used as a starter for cheese and also widely used in the production of lactic acid drinks, yogurt, and the like.

The filtrate of the lactic acid bacterial culture used in the present invention can be obtained by culturing the above-mentioned lactic acid bacteria, removing the solid components left after crushing the bacterial cells of the lactic acid bacteria in the culture medium obtained, i.e., the residue of the bacterial cells left after making the cell wall portion into a colloid or dissolving it.

The lactic acid bacterial culture can be produced under any conditions, as long as they permit satisfactory multiplication of the lactic acid bacteria. The composition of culture medium, culturing temperature, culturing pH, culturing time, etc. may be varied, depending on the type of the lactic acid bacteria used. The culturing conditions and culturing method, for instance, can be selected from those given in Bergey's Manual of Determinative Bacteriology (8^{th} Edition, 1974) and those given in the user's manuals provided by the manufacturers of commercially available lactic acid bacteria.

The medium can be prepared, for example, by combining one or more carbon sources and nitrogen sources such as whey glucose, and peptone. The culturing temperature can be varied, depending on the type of bacterium used. The normally used culturing temperature (approx. 20-45°C) is suitable. The culturing can be done at an even higher temperature in the case of heat-resistant lactic acid bacteria. The bacteria grow quite well in the pH range 2-4. However, the culturing pH of the initial medium may be neutral or weakly alkaline.

The culturing may be done for a period of a few hours to 72 hours, preferably for about 15-50 hours. The amount of lactic acid bacterium culture to be added per liter of the medium is 10-100 ml.

A set of the culturing conditions is given below, as an example. But this in no way limits the scope of the invention.

When the lactic acid bacteria belonging to the genus Streptococcus are used, the culturing can be done in the MRS medium (available from Difco, Co.) at 30-37°C and pH 6.8, for 24-48 hours. In the case of the lactic acid bacteria belonging to the genus Leuconostoc also, culturing can be done in the MRS medium at 30-37°C, and pH 6.8, for 24-48 hours. For the lactic acid bacteria belonging to the genus Pediococcus also, culturing can be done in the MRS medium at 30-37°C and pH 6.8, for 24-48 hours.

In the case of the lactic acid bacteria belonging to the genus Lactobacillus also, culturing can be done in the MRS medium at 30-37°C and pH 6.8, for 24-48 hours.

For the lactic acid bacteria belonging to the genus Bifidobacterium, the culturing may be done in BL or EG medium under anaerobic conditions at 30-37°C and pH 7-8, for 24-48 hours.

In a specific example of the present invention, a medium containing whey and glucose (10% and 2%, respectively, for example) is inoculated with a lactic acid bacterium, and stationary culturing is done at 35-37°C for 48 hours.

After culturing the lactic acid bacteria, the bacterial cells are crushed, the culture suspension is filtered or centrifuged to remove the residue of bacterial cells, and the filtrate of cultures is recovered.

The compositions of the media described above are given below.

### MRS medium (for 1 liter of medium)

| | |
|---|---|
| Peptone (Oxoid) | 10 g |
| Meat extract | 10 g |
| Yeast extract | 5 g |
| K₂HPO₄ | 2 g |
| Diammonium hydrogen citrate | 2 g |
| Glucose | 20 g |
| Tween 80 | 1 g |
| Sodium acetate | 5 g |
| MgSO₄·7H₂O | 0.58 g |
| MnSO₄·4H₂O | 0.28 g |

(The volume was made up to 1 liter with purified water, pH 6.2-6.6, sterilized for 15 minutes at 121°C)

### BL (Agar) medium

| | |
|---|---|
| Beef extract (Oxoid) | 3 g |
| Proteose peptone No. 3 (Difco) | 10 g |
| Trypticase (BBL) | 5 g |
| Phytone (BBL) | 3 g |
| Yeast extract (Difco) | 5 g |
| Liver extract | 150 ml |
| Glucose | 10 g |
| Soluble starch | 0.5 g |
| Solution A | 10 ml |
| Solution B | 5 ml |
| Tween 80 | 1 g |
| Bactoagar (Difco) | 15 g |
| L-Cysteine hydrochloride (5% solution) | 10 ml |
| Horse blood | 50 ml |
| Purified water pH 7.2 | 765 ml |

Solution A: 25 g of K₂HPO₄ and 25 g of K₂HPO₄ are dissolved in 250 ml of distilled water.
Solution B: 10 g of MgSO₄·7H₂O, 0.5 g of FeSO₄·7H₂O, 0.5 g of NaCl, and 0.337 g of MnSO₄ are dissolved in 250 ml of purified water.
Liver extract: 10 g of liver powder (Kyokuto) is extracted for about one hour in 170 ml of purified water placed in a 50-60°C warm water bath. It is heated for several minutes at 100°C and filtered through filter paper after adjusting the pH thereof to 7.2.
Preparation of medium: The components other than the L-cysteine hydrochloride and horse blood are heated to dissolve them. Then, after adjusting the pH, the solution is sterilized for 20 minutes at 115°C. The solution is then cooled to 50°C, the cysteine hydrochloride and horse blood are added to prepare flats.

### EG (EUGON) medium

| | |
|---|---|
| Bactotryptose (Difco) | 1.5% |
| Bactosoeton (Difco) | 0.5% |
| Bactodextrose (Difco) | 0.55% |
| L-Cysteine (Difco) | 0.07% |
| NaCl | 0.4% |
| Na₂SO₃ | 0.02% |
| Purified water to make | 100% |

The hair growth tonic of the present invention contains the filtrate of a lactic acid bacterial culture thus obtained as its active component. The amount of the filtrate of cultures in the composition is as much as would give a suitable effect on hair growth, and is not particularly limited. It can, for example, be 0.1% or more, preferably 0.1-20% (% by volume in all cases). The filtrate of the lactic acid bacterial culture of the present invention can be concentrated to a suitable level, such as 2-10 times, preferably 3-5 times, by employing an ordinary method of concentration, before adding to the hair growth tonic as the active ingredient.

Lactic acid bacteria are known to synthesize vitamins of the B group inside human guts, activate leukocytes, enhance the bacterial infection preventing activity by strengthening the immune system, and produce antibacterial substances that exclude harmful bacteria. They are also known to adsorb carcinogens and enable their excretion through human stools, and to have a blood cholesterol lowering effect. Through the present invention, it has now become clear that the filtrate of cultures thereof have hair growth promoting and hair loss prevention effect. Some components contained in the filtrates of lactic acid bacterial cultures have the effect of promoting blood flow to the terminal capillaries, promoting of the division and multiplication of hair matrix cells, and activation of hair roots which improves the lowered functions of the hair matrix cells. Which components are effective is not very clear. However, it is assumed that various components produced by lactic acid bacteria, which are present in the filtrate of lactic acid bacterial cultures, act synergistically when supplied to the hair papilla and exert a hair growth promoting effect on the hair matrix cells.

In the hair growth tonic of the present invention, the above-described culture filtrate can be used singly. However, for the sake of ease in use, it is preferable to use it along with carriers such as excipients and diluents permitted in drugs (including quasi drugs, used with this meaning hereinafter) and cosmetics. Examples of such carriers include purified water, ordinary water, physiological saline, ethanol, etc., all of which are normally used in hair growth tonics.

The hair growth tonic of the present invention can contain other known hair growth promoting components. Examples of such components include activators of living cells (for example laurel extract, chlorophyll, etc.); blood flow promoters (such as alder fly extract, cephalanthin, Vitamin E and its derivatives, γ-orizanol, etc) and local stimulants (such as capsicum tincture, ginger tincture, cantharis tincture, benzyl ester of nicotinic acid, etc) that promote blood flow to the peripheral capillaries; nutrient preparation that supply nutrients (for example, Vitamins A, B₁, B₂, B₆, E and their derivatives, pantothenic acid and its derivatives, vitamins such as biotin, amino acids such as cystine, cysteine, methionine, leucine, tryptophan, amino acid extracts) to the area surrounding the hair matrix cells; female hormones (for example, estradiol, ethynylestradiol, etc) which have antagonistic action against the male hormone; hair root activators (such as pantothenic acid and its derivatives, placenta extract, allantoin, Kanko-so No. 301, etc) for improving the lowered function of the hair matrix cells; moisturizers (such as glycerol and pyrrolidone carboxylic acids) to prevent drying of the scalp, etc.

The hair growth tonic of the present invention can also contain drugs for preventing dandruff and itchiness. Examples of such drugs include keratin exfoliant-resolvents such as salicylic acid, sulfur, resorcinol, and selenium sulfide; anti-seborrhea agents such as pyridoxine and its derivatives; disinfectants such as pyrethion zinc, trichlorocarbanilide/acetic acid tocophenol, benzalkonium chloride, benzethonium chloride, chlorohexidine, and hinoki (Japanese cypress) thiol; antiinflammatory agents such as glycyrrhetic acid and its derivatives, hydrocortisone acetate, and prednisolone; antipruritic agent such as diphenhydramine hydrochloride, maleic acid chlorophenylamine, camphor, and dl-or l-menthol. In addition to these, it can contain suitable amounts of components described in *Keshohin Betsu Kyokakijun* (Permissible Standards for Cosmetics) (Yakuji Nopposha) such as perfumes, fresheners, emulsifiers, solubilizing agents, pH regulators, and coloring agents.

The hair growth tonic of the present invention can be used as a drug as well as a cosmetic. Its effects as a drug are the improvement and prevention of circular alopecia areata, premature alopecia, and male pattern alopecia. It is also effective in promoting hair revival, hair regeneration, hair growth, and hair nourishment, and in preventing falling of hair. Because of this, the hair growth tonic of the present invention can be used effectively as a cosmetic. The lactic acid bacteria used in the present invention are used in the preparation of food or are naturally present in food. Thus, the bacterial culture medium has a very low toxicity.

The hair growth tonic may be in the form of a liquid formulation, emulsion, cream, lotion, gel, liquid, foam, spray, etc. It can also be compounded with shampoos, rinses, and treatments. For example, oil and fats (such as olive oil, and triglyceride), hydrocarbons (such as liquid paraffin, vaseline, and yellow beeswax), higher fatty acids, higher fatty acid esters, higher alcohols, surfactants, tackifiers, chelating agents, and the like may be used to prepare a cream. To prepare a gel, water soluble polymers (such as carboxyvinyl polymer, and methyl cellulose), setting agents (such as polyvinylpyrrolidone), alkalies, surfactants, chelating agents, and the like may be used. If it is in the form of a spray or foam, atomizing agents (such as dimetyl ether, and liquefied petroleum gas) may be used. Gums (such as gum tragacanth, and gum karaya), polymers (such as polyvinylpyrrolidone/vinyl acetate copolymer), and the like may be used in the case of lotions.

The hair growth tonic of the present invention may be applied (for example, by spraying 1 ml) on the scalp at least once a day, preferably twice or more often, and the scalp may be stimulated by tapping or massaging to achieve a significant hair growth promoting effect.

The hair growth tonic of the present invention will now be explained in greater detail referring to some examples. However, the scope of the invention is not limited by these examples.

### [Example 1] Preparation of hair growth tonic containing filtrate of a Streptococcus lactis culture

A medium prepared by adding 10% of whey and 2% of glucose to 1 liter of sterilized water was inoculated with 50 ml of Streptococcus lactis. It was then subjected to stationary culture for 48 hours at 35°C. After culturing, the cell walls were crushed, made into a colloid and dissolved. This bacterial suspension was centrifuged to remove the bacterial cell residues. The culture filtrate thus obtained was used in the preparation of the following examples of the hair growth tonic (the quantities are expressed in % by volume).

### Formulation 1

| | |
|---|---|
| Filtrate of Streptococcus lactis culture | 1.5% |
| Pure water to make up | 100% |

### Formulation 2

| | |
|---|---|
| Filtrate of Streptococcus lactis culture | 1.5% |
| Laurel extract | 0.5% |
| Ethanol | 15% |
| Pure water to make up | 100% |

### Formulation 3

| | |
|---|---|
| Filtrate of Streptococcus lactis culture | 1.5% |
| Capsicum tincture | 0.1% |
| dl-Menthol | 0.1% |
| Water-soluble placenta extract | 0.1% |
| Laurel extract | 0.5% |
| Ethanol | 15% |
| Pure water to make up | 100% |

### [Example 2] Preparation of hair growth tonic containing filtrate of Lactobacillus bulgaricus culture

A medium prepared by adding 10% of whey and 2% of glucose to 1 liter of sterilized water was inoculated with 50 ml of Lactobacillus bulgaricus. It was then subjected to stationary culture for 48 hours at 35°C. After culturing, the cell walls were crushed, made into a colloid and dissolved. This bacterial suspension was centrifuged to remove the bacterial cell residues. The culture filtrate thus obtained was used in the preparation of the following examples of the hair growth tonic (the quantities are expressed in % by volume).

### Formulation 4

| | |
|---|---|
| Filtrate of Lactobacillus bulgaricus culture | 1.5% |
| Purified water to make up | 100% |

### Formulation 5

| | |
|---|---|
| Filtrate of Lactobacillus bulgaricus culture | 1.5% |
| Laurel extract | 0.5% |
| Ethanol | 15% |
| Purified water to make up | 100% |

### Formulation 6

| | |
|---|---|
| Filtrate of Lactobacillus bulgaricus culture | 1.5% |
| Capsicum tincture | 0.1% |
| dl-Menthol | 0.1% |
| Water-soluble placenta extract | 0.1% |
| Laurel extract | 0.5% |
| Ethanol | 15% |
| Purified water to make | 100% |

### [Example 3] Hair growth test and results

A suitable amount of the hair growth tonic of Example 1 (Formulation 2) was applied twice a day, morning and evening, on the scalp of three alopecic males. The effect of 4 or 5 months of treatment was evaluated visually. Significant effect on hair growth occurred in all the cases (refer to Fig. 1). Similar positive effect on hair growth was confirmed with the hair growth tonic of Example 2 (Formulation 5) when applied on three alopecic men, for 2-3 months. The results are shown in Table 1 (for Formulation 2) and Table 2 (for Formulation 5).

**[Table 1]**

| Test subject | Sex & Age | Duration of use | Effect |
|---|---|---|---|
| A | Male, in his 40's | 4 months | ++ |
| B | Male, in his 40's | 5 months | +++ |
| C | Male, in his 40's | 5 months | ++ |

**[Table 2]**

| Test subject | Sex & Age | Duration of use | Effect |
|---|---|---|---|
| D | Male, in his 50's | 3 months | ++ |
| E | Male, in his 50's | 3 months | ++ |
| F | Male, in his 60's | 2 months | + |

### Meaning of symbols

- : Worsened
0 : No visible effect
+ : No visible effect, but positive effect could be confirmed
by microscopic observation
++ : Effect was visible
+++ : Very significant effect could be seen

The above examples show that the hair growth tonic of the present invention is effectively used in prevention of hair loss and its treatment.

### [Example 4] Preparation of hair growth tonic and hair growth test

A hair growth tonic having the composition shown below was prepared using the Streptococcus lactis culture filtrate prepared in Example 1. The tonic was applied on three males. The duration of usage thereof and the results obtained are given below under (1)-(3).

### Formulation 7

| | |
|---|---|
| Filtrate of Streptococcus lactis | 2% |
| Purified water to make us | 100% |

### (1) Male I (50 years old)

The subject was bald on the back of his head. This baldness was so extensive that the even the hair whirl at the top of the head could not be seen. Starting from October 1999 about 5 cc of the hair growth tonic prepared according to the Formulation 7 was sprayed daily on the back of his head, for about 3 months. His hair growth recovered almost to the level of the surrounding area.

### (2) Male G (65 years old)

His head was as bald as an egg. When about 5 cc of the above-mentioned hair growth tonic was applied daily, for about 2 years starting from January 1998, hair growth recovered to the level of a close cropped head of hair.

### (3) Male K (60 years old)

He was not bald. When about 3 cc of the tonic was applied daily for 10 days starting from January 10, 2000, the falling of hair and dandruff disappeared.

### INDUSTRIAL APPLICABILITY

As discussed above, the hair growth tonic according to the present invention 1effectively promotes hair growth. It is useful as a drug and cosmetic that can prevent hair loss.

## Claims

1. A hair growth tonic containing a filtrate of lactic acid bacterial culture as an active ingredient.

2. The hair growth tonic according to claim 1, characterized in that the lactic acid bacterium is a bacterium belonging to Streptococcus or Lactobacillus.

3. The hair growth tonic according to claim 2, characterized in that the lactic acid bacterium of Streptococcus is Streptococcus lactis.

4. The hair growth tonic according to claim 2, characterized in that the lactic acid bacterium of Lactobacillus is Lactobacillus bulgaricus.

5. The hair growth tonic according to claim 2, characterized by further comprising other components known to promote hair growth.

6. The hair growth tonic according to claim 5, characterized in that the other components known to promote hair growth are laurel extract and/or chlorophyll.
